# EUROPEAN PATENT APPLICATION

(11) **EP 4 070 798 A1**
(43) Date of publication of application: **12.10.2022**
(21) Application number: 20895228.3
(22) Date of filing: 29.09.2020
(51) Int. Cl.: A61K 31/7032, A61K 31/045, A61K 31/555, A61P 17/02, A61P 3/10

(54) **CHRONIC WOUND HEALING COMPOSITION AND APPLICATION THEREOF**

(30) Priority: 06.12.2019 CN 201911241631
(71) Applicant: Lo, Hsu En, Taipei City 114 (TW)
(72) Inventor: Lo, Hsu En, Taipei City 114 (TW)
(74) Representative: Straus, Alexander
(86) International application number: PCT/CN2020/118933
(87) International publication number: WO 2021/109704

(57) **Abstract**

A composition having a chronic wound healing efficacy, particularly diabetic wound healing, mainly composed of the following three components: (1) an anti-inflammatory agent selected from the group consisting of acteoside, isoacteoside and a combination thereof, (2) an astringent selected from the group consisting of gallic acid, subgallic acid, their salts and a combination thereof, and (3) a cooling agent selected from the group consisting of borneal, menthol and a combination thereof, and optionally combined with one or more pharmaceutically acceptable carriers. The present invention also provides use of said composition in the preparation of a medicament for treating a chronic wound, particularly a diabetic wound.

## Description

### BACKGROUND OF THE INVENTION

### FIELD OF THE INVENTION

The present invention relates to a composition with chronic wound healing efficacy and applications thereof.

### DESCRIPTION OF THE PRIOR ART

With the aging of the human population, chronic diseases have become a focus of health care, and the care of chronic wounds is a very important part. Chronic wounds refer to wounds that cannot achieve normal wound healing in structure and appearance after proper treatment for a predetermined period of time, and there is still no effective therapeutic agents so far. Types of chronic wounds include bedsores or pressure ulcers caused by long-term bed rest, wound ulcers in diabetic patients, limb gangrene caused by peripheral vascular obstruction, ulcers caused by arterial or venous obstruction, non-healing wounds in cancer patients, and chronic poor healing caused by acute wound infections, etc.

Acteoside, an active ingredient in Chinese herbal medicine, has lipase inhibitory effects and is known to have antibacterial, anti-inflammatory, antiviral, antioxidant, and neuroprotective activities.

Acteosides have been reported as potent antimicrobial constituents and are known to have good antibacterial efficacy against *Staphylococcus aureus* (Guillermo et al., 1999, Journal of Ethnopharmacology 66(1):75-8). Acteosides also have good anti-inflammatory effects (Speranza et al., 2009, Journal of biological regulators and homeostatic agents 23(3): 189-95). In addition, Chinese Patent No. 103816169B issued on January 6, 2016 disclosed that acteoside can be used to prepare and prevent vascular dementia, and has protective and therapeutic effects on cellular ischemia and hypoxia caused by low glucose and hypoxia. Also, Taiwan Patent Application No. 201601739 published on January 16, 2016 provides a preparation method of an osmanthus flower extract. The main active ingredient is acteoside, which has antioxidant, skin care, anti-microbial and anti-inflammatory effects. However, there are no reports that it can be used in chronic wound healing.

### SUMMARY OF THE INVENTION

The present invention provides a novel composition with efficacies of chronic wound healing, mainly composed of the following three components: (1) an anti-inflammatory agent selected from the group consisting of an acteoside, an isoacteoside and a combination thereof, (2) an astringent selected from the group consisting of a gallic acid, a subgallic acid, their salts and a combination thereof, and (3) a cooling agent selected from the group consisting of a borneal, a menthol and a combination thereof and optionally combined with one or more pharmaceutically acceptable carriers.

In another aspect, the present invention provides use of said composition in the preparation of a medicament for treating a chronic wound.

According to an embodiment of the present invention, the chronic wound refers to a diabetic wound.

According to an embodiment of the present invention, an equivalent ratio of the three components (1) the anti-inflammatory agent: (2) the astringent: (3) the cooling agent is 1:0.1-10:0.1-10.

According to a preferred embodiment of the present invention, an equivalent ratio of the three components (1) the anti-inflammatory agent: (2) the astringent: (3) the cooling agent is 1:0.5-5:0.5-5.

According to a specific embodiment of the present invention, an equivalent ratio of the three components (1) the anti-inflammatory agent: (2) the astringent: (3) the cooling agent is about 1:1:1.

According to an embodiment of the present invention, the anti-inflammatory agent is acteoside having the structure of Formula I below or an isomer thereof:

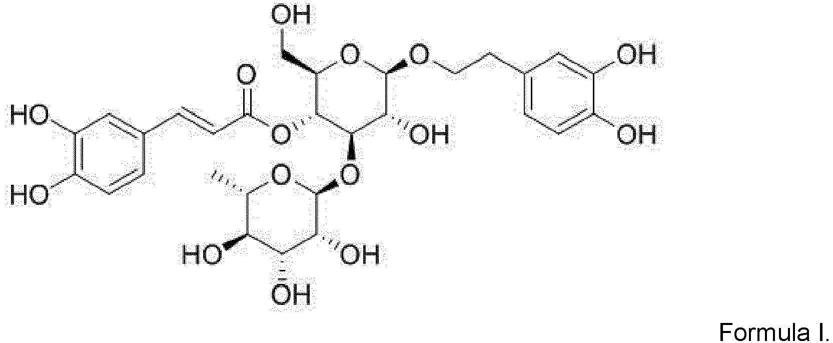

According to an embodiment of the present invention, the astringent is a gallic acid having the structure of Formula II below:

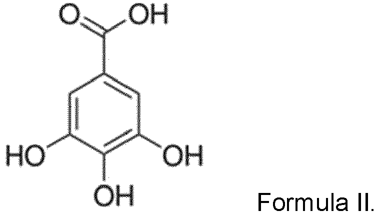

According to an embodiment of the present invention, the astringent is a salt of gallic acid or subgallic acid, preferably a bismuth salt.

According to a best embodiment of the present invention, the astringent is a bismuth subgallate having the structure of Formula IIa below:

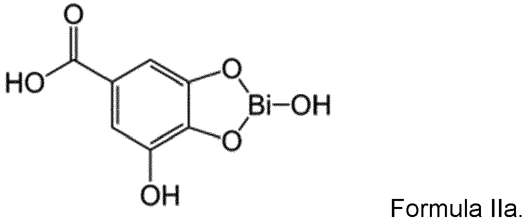

According to an embodiment of the present invention, the cooling agent is a borneal having the structure of Formula IIIa below:

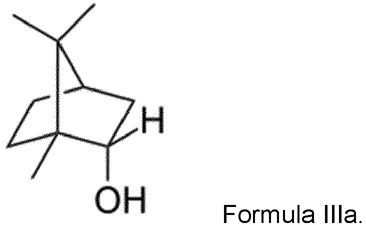

According to an embodiment of the present invention, the cooling agent is a menthol having the structure of Formula IIIb below:

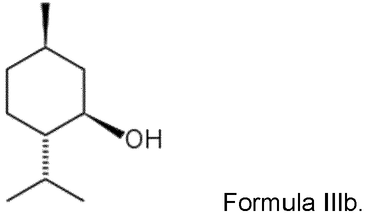

In a further aspect, the present invention provides a pharmaceutical composition with efficacies of chronic wound healing, mainly composed of the following three components: (1) acteoside, (2) bismuth subgallate, and (3) borneal, and optionally combined with one or more pharmaceutically acceptable carriers.

According to the present invention, an equivalent ratio of the three components (1) acteoside: (2) bismuth subgallate: (3) borneal in the pharmaceutical composition is 1:0.1-10:0.1-10; preferably 1:0.5-5:0.5-5.

According to an embodiment of the present invention, the equivalent ratio of the three components (1) acteoside: (2) bismuth subgallate: (3) borneal in the pharmaceutical composition is about 1:1:1.

According to the present invention, the pharmaceutical composition comprises (1) 0.05%-10% of acteoside, (2) 0.05%-10% of bismuth subgallate, (3) 0.02%-5% of borneal by weight and a pharmaceutically acceptable carrier.

These and other aspects of the present invention will become more apparent from the following description and drawings of preferred embodiments. Although there may be changes or modifications therein, they do not depart from the spirit and scope of the novel concepts disclosed in the present invention.

### BRIEF DESCRIPTION OF THE DRAWINGS

The foregoing description and the embodiments can be better illustrated by the accompanying drawings. In order to enhance the description of the present invention, suitable drawings of embodiments are listed herein.

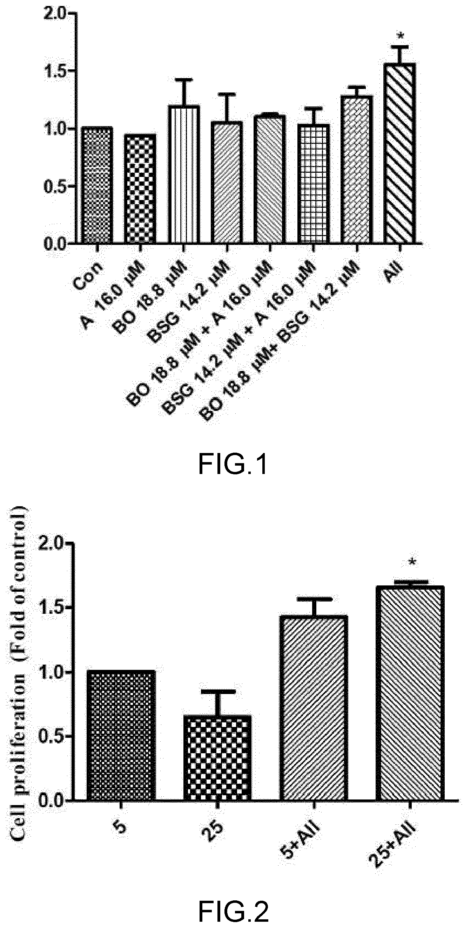

FIG. 1 shows the results of promoting cell proliferation of HaCaT cells cultured in a high glucose (20 mM) environment for 48 hours by the composition of the present invention (Con: control group; A 16.0 µM; BO 18.8 µM; BSG 14.2 µM; BO 18.8 µM + A 16.0 µM; BSG 14.2 µM + A 16.0 µM; BO 18.8 µM + BSG 14.2 µM; All (A 16.0 µM + BO 18.8 µM + BSG 14.2 µM); * indicates significant difference, p<0.05).
FIG. 2 shows the comparison of the composition of the present invention (All, A 16.0 µM + BO 18.8 µM + BSG 14.2 µM) against the cell proliferation of HaCaT cells cultured in low glucose (5 mM) and high glucose (25 mM) environments for 48 hours, * indicates significant difference, p <0.05 (5: 5mM glucose; 25: 25mM glucose; 5+AII: 5mM glucose with the addition of the composition of the present invention (A 16.0 µM + BO 18.8 µM + BSG 14.2 µM); 25+AII: 25 mM glucose with the addition of the composition of the present invention (A 16.0 µM + BO 18.8 µM + BSG 14.2 µM).

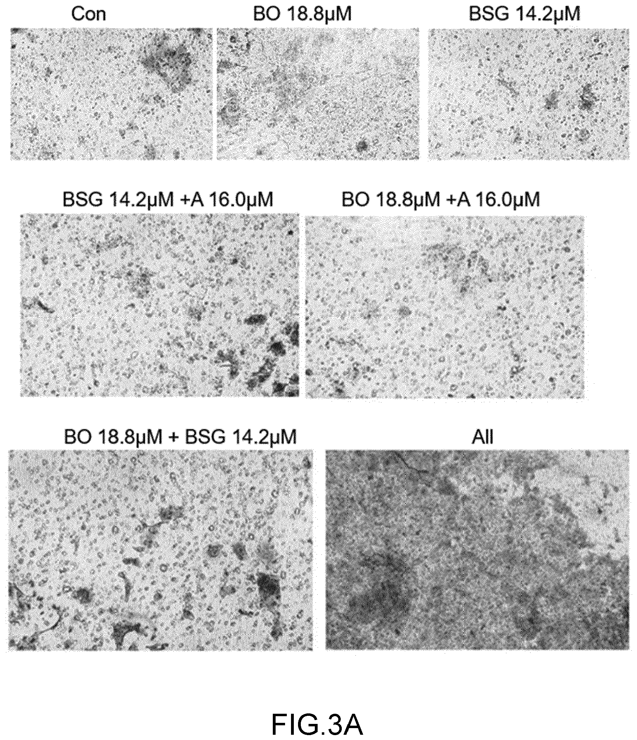

FIG. 3A shows the image of the cell migration of HaCaT cells cultured in the high glucose (25mM) environment with the composition of the present invention (All) and the control group for 48 hours (Con: control group; BO 18.8 µM; BSG 14.2 µM; BO 18.8 µM + A 16.0 µM ; BSG 14.2 µM + A 16.0 µM; BO 18.8 µM + BSG 14.2 µM; All (A 16.0 µM + BO 18.8 µM + BSG 14.2 µM).

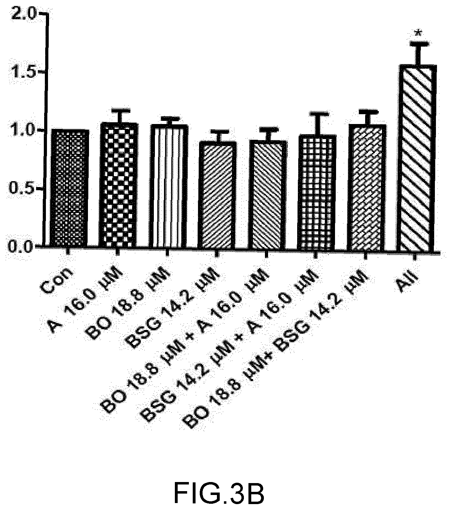

FIG. 3B shows the comparison between the composition of the present invention (All) and the control group when HaCaT cells are cultured in a high glucose (25mM) environment for 48 hours, and the cell proliferation ratio of the opposite group is 1.0 compared to other experimental groups (Con: control group; A 16.0 µM; BO 18.8 µM; BSG 14.2 µM; BO 18.8 µM + A 16.0 µM; BSG 14.2 µM + A 16.0 µM; BO 18.8 µM + BSG 14.2 µM; All (A 16.0µM + BO 18.8µM + BSG 14.2µM)).

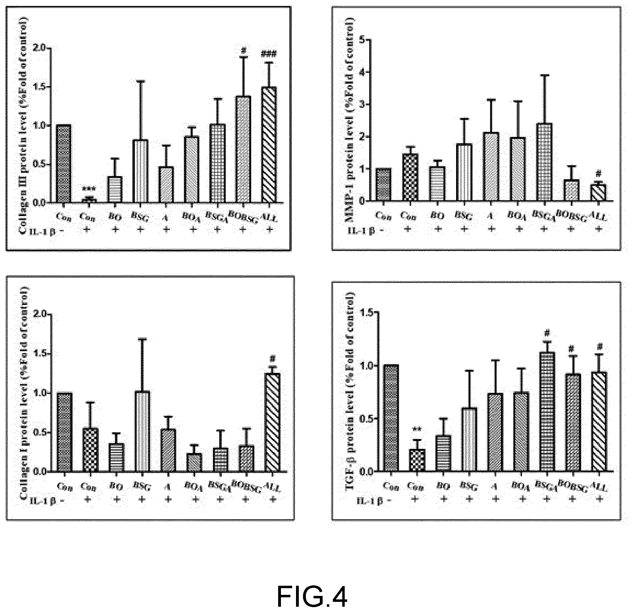

FIG. 4 shows the comparison between the composition of the present invention and the control group on HDF cells treated with IL-1β to determine the changes in the concentrations of Collagen III, MMP-1, Collagen I and TFG-β proteins.

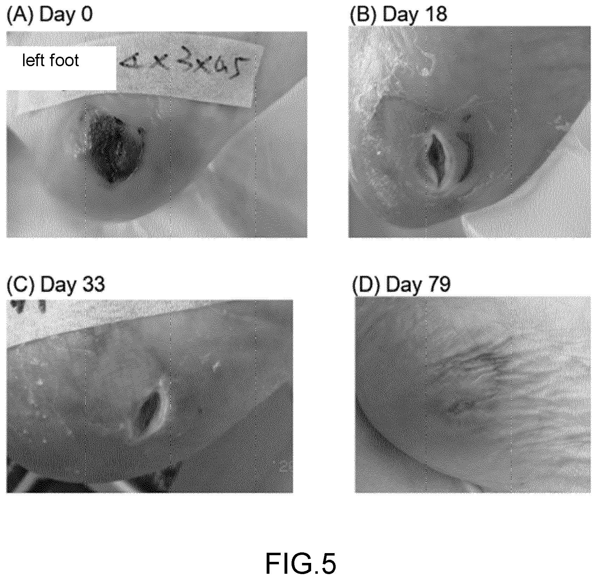

FIG. 5 shows the wound healing process of case 1 (diabetic and stroke patient, male, 77 years old), the wound size was 4 cm × 3 cm × 0.5 cm on day 0 (A), the wound started to close obviously on day 18 (B), on day 33 the wound was 1.5 cm × 0.5 cm × 0.2 cm (C), and the wound healed completely on day 79 (D).

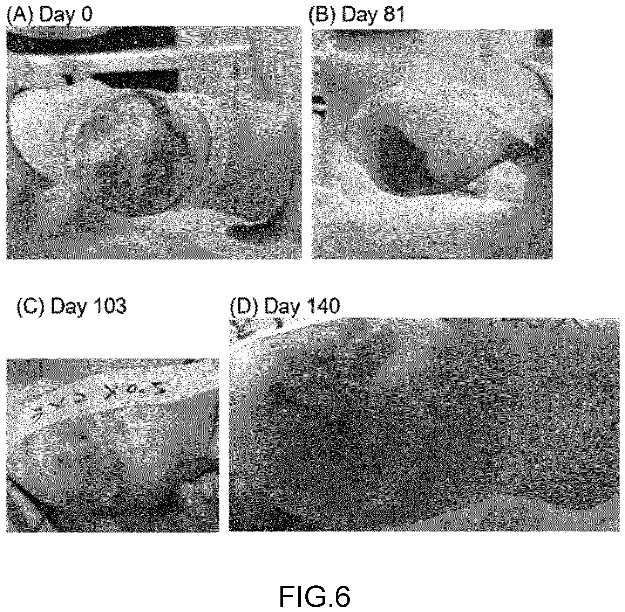

FIG. 6 shows the wound healing process of case 2 (diabetic and stroke patient, male, 77 years old), the wound size was 15cm × 11 cm × 2 cm on day 0 (A), the wound size on day 75 was 5.5cm × 11 cm × 1 cm (B), the wound was 3 cm × 2 cm × 0.5 cm on day 103 (C), and the wound was completely healed on day 140 (D).

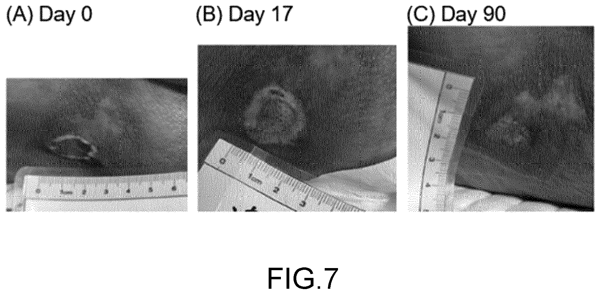

FIG. 7 shows case 3 (diabetic and lung cancer patient, female, 82 years old), the wound size was 2.5cm × 1 cm × 0.5 cm on day 0 (A), the wound size on day 17 was 1.5cm × 1 cm × 0.2 cm (B), and the wound healed completely on day 90 (C).

### DETAILED DESCRIPTION OF THE EMBODIMENTS

The terms used in the description of the present invention generally have their original meanings in the technical field, in the summery of the present invention, and in the specific context in which each term is used.

The term "a" as used herein, unless otherwise specified, refers to the quantity of at least one (one or more).

As used herein, the term "chronic wound" refers to a wound that fails to achieve normal wound healing in structure and appearance after proper treatment for a predetermined period of time. Types of chronic wounds include bedsores or pressure ulcers caused by long-term bed rest, diabetic wound ulcers, limb gangrene caused by peripheral vascular obstruction, ulcers caused by arterial or venous obstruction, non-healing wounds in cancer patients, and chronic poor healing caused by acute wound infection. According to the present invention, the composition of the present invention is shown to have a healing effect on dermatological wounds of chronic wounds, especially diabetic wounds.

As used herein, the term "treatment" includes the meaning of "treating" or "promoting" and means improving symptoms.

As used herein, the term "patient" includes humans and animals, especially mammals.

As used herein, the term "pharmaceutically acceptable carrier" refers to diluents, excipients and the like which are commonly used in the manufacture of pharmaceutical compositions by techniques commonly used in medicine. According to the present invention, it can be formulated into the form of medicine, cosmetic or medical material. According to the present invention, it can be applied in the form of a topical manner, for example, in the form of a spray. Spray forms include sprays and liquids; or semi-solid or solid forms, preferably solid forms having a dynamic viscosity greater than that of water. Suitable formulations include, but are not limited to, suspensions, emulsions, creams, ointments, liniments, and the like. Preferably, it is in the form of an ointment. Regardless of the form, the pharmaceutical compositions of the present invention may also contain emollients, fragrances or pigments to enhance their acceptability for various uses.

As used herein, the term "therapeutically effective amount" means an amount effective to treat a wound in the management of symptoms. Appropriate doses can be used according to the needs of patients or wounds, according to commonly used techniques or clinical knowledge in medicine, and can be adjusted according to the mode of administration and the situation of treatment, including age, weight, symptoms, treatment effect, mode of administration and treatment time.

The present invention provides a composition having a chronic wound healing efficacy, mainly composed of the following three components: (1) an anti-inflammatory agent selected from the group consisting of acteoside, isoacteoside and a combination thereof, (2) an astringent selected from the group consisting of gallic acid, subgallic acid, their salts and a combination thereof, and (3) a cooling agent selected from the group consisting of borneal, menthol and a combination thereof, and optionally combined with one or more pharmaceutically acceptable carriers. The present invention also provides use of said composition in the preparation of a medicament for treating a chronic wound, particularly a diabetic wound.

According to embodiments of the present invention, the composition of the present invention has a significant enhancing effect on cell proliferation and migration in a high-glucose environment culture, but a single component or any combinations of two has no effect. In particular, the composition of the present invention has relatively ineffective for culture under a low-glucose environment, which shows the special effect of the composition of the present invention on the healing of diabetic wounds, and can further support its effect on chronic wound healing.

According to the present invention, the composition with an appropriate content can be prepared according to general conventional techniques, wherein the examples may include an equivalent range of each ingredient:
(1) the anti-inflammatory agent: 1;
(2) the astringent: 0.1-10; preferably 0.5-5.
(3) the cooling agent: 0.1-10; preferably 0.5-5.

According to a specific embodiment of the present invention, the equivalent ratio of the three components of (1) the anti-inflammatory agent: (2) the astringent: (3) the cooling agent is about 1:1:1.

The subject of the present invention is to provide a pharmaceutical composition with the effect of treating chronic wound healing, mainly composed of the following three components: (1) acteoside, (2) bismuth subgallate, and (3) borneal, and is optionally combined with one or more pharmaceutically acceptable carriers, such as a product with a trademark of NuDFC7 ^{™}, which is not commercially available yet.

According to the present invention, an equivalent ratio of the three components (1) the anti-inflammatory agent: (2) bismuth subgallate: (3) borneal in the pharmaceutical composition is 1:0.1-10:0.1-10; preferably 1:0.5-5:0.5-5, of which a specific embodiment is about 1:1:1.

According to the present invention, the pharmaceutical composition comprises (1) 0.05%-10% of acteoside, (2) 0.05%-10% of bismuth subgallate, (3) 0.02%-5% of borneal by weight and the pharmaceutically acceptable carrier.

According to an embodiment of the present invention, the pharmaceutical composition comprises (1) 0.1%-5% of acteoside, (2) 0.06%-6% of bismuth subgallate, (3) 0.03%-3% of borneal by weight and the pharmaceutically acceptable carrier.

According to a preferred embodiment of the present invention, the pharmaceutical composition comprises (1) 0.3%-5% of acteoside, (2) 0.5%-5% of bismuth subgallate, (3) 0.5%-1% of borneal and the pharmaceutically acceptable carrier.

According to the present invention, the composition may comprise a suitable excipient for the preparation of the composition with an external pharmaceutical form, a cosmetic form or a medicinal material form.

According to the present invention, the composition may further include a therapeutic agent, such as other anti-inflammatory agents, antibacterial agents or other therapeutic agents.

The foregoing description of the invention and the following examples illustrate the present invention, but are not intended to limit the scope of the invention.

### Examples

Medical preparation:
(1) Acteoside (with A as the code): purchased from Sigma-Aldrich in the United States, USA (St. Louis, MO, USA).
(2) Bismuth subgallate (with BSG as the code): Sigma-Aldrich in the United States (St. Louis, MO, USA).
(3) Borneal (with BO as the code): Sigma-Aldrich in the United States (St. Louis, MO, USA).

According to the present invention, the compositions may be prepared with the following ingredients:

| Composition | The anti-inflammat ory agent | The astringent | The cooling agent |
|---|---|---|---|
| Range of equivalent ratio | 1 | 0.1-10 | 0.1-10 |
| Specific component | Acteoside | Bismuth subgallate | Borneal |
| Range of weight percentage | 0.05%-10% | 0.05%-10% | 0.02%-5% |
| Composition preparation example 1 (weight percentage) | 0.05% | 0.025% | 0.02% |
| Composition preparation example 2 (weight percentage) | 0.3% | 0.5% | 0.5% |
| Composition preparation example 3 (weight percentage) | 5% | 5% | 1% |
| Composition preparation example 4 (weight percentage) | 1% | 4% | 0.1% |
| Composition preparation example 5 (weight percentage) | 10% | 1% | 0.05% |

The content of each component of the composition of the present invention for the following cell experiments is:
(1) Acteoside (A) 16.0µM (0.01µg/µl), i.e. 0.1%;
(2) Bismuth subgallate (BSG)14.2µM (0.0056µg/µl), i.e. 0.056%; and
(3) Borneal (BO) 18.8µM (0.0029µg/µl), i.e. 0.029%.

### Cell culture:

Human dermal fibroblasts (HDF cells) or human keratinocytes (HuCaT cells) were cultured in a humid incubator at 37°C in an environment of 5% carbon dioxide and 95% oxygen and placed in Dulbecco's modified Eagle's medium (DMEM) supplemented with glucose, 10% fetal bovine serum, 100 IU/mL penicillin, 100 µg/mL streptomycin, 2 mM sodium pyruvate and 1% nonessential amino acids (NEAA). The medium was changed every two days. For subculture, cells were harvested and re-cultured at a 1:10 dilution after exposure to 0.1% Trypsin-EDTA mixture. The cells used in the study ranged from 12 to 40 passages. 24 hours before drug treatment, cells were seeded and left for 24 hours. The control group was maintained in the medium and supplemented with drugs. Under this culture condition, cell growth and differentiation are not affected.

### Example 1 Cell Proliferation Assay

HaCaT cells were cultured at 3 × 10⁵ with standard addition of 25 mM glucose in a 12-well culture dish for 48 hours. Cell growth and viability were analyzed with the CCK-8 reagent kit (Dojindo Molecular Technologies, Kumamoto, Japan). 5 µL of CCK-8 solution was added to each well and incubated at 37°C for one hour, then the absorbance at 450 nm was measured using a spectrophotometer Microplate reader (SpectraMax, Molecular Device, USA). The experimental results were analyzed by comparing the values of the samples with that of the control group. The tested experimental groups include:
(1) Con: the control group;
(2) A 16.0µM;
(3) BO 18.8µM;
(4) BSG 14.2µM;
(5) BO 18.8µM +A 16.0µM;
(6) BSG 14.2µM +A 16.0µM;
(7) BO 18.8µM + BSG 14.2µM;
(8) All (A 16.0µM + BO 18.8µM + BSG 14.2µM).

The results of cell proliferation after 48 hours of culture are shown in FIG. 1, which shows that the composition of the present invention (All; A 16.0µM + BO 18.8µM + BSG 14.2µM) is the best, providing an unexpectedly significant effect of promoting cell proliferation (p < 0.05), but a single component is ineffective, or a combination of any two is less effective than a combination of the three.

### Example 2 Comparison of cell culture analysis in low-glucose and high-glucose environments (Comparison in Cell Proliferation Assay)

HaCaT cells were cultured at 3 × 10⁵ with addition of 5 mM and 25 mM glucose in a 12-well culture dish for 48 hours, and the cell proliferation was observed with addition of the composition of the present invention (All: A 16.0 µM + BO 18.8 µM + BSG 14.2 µM).

The results of cell proliferation after 48 hours of culture are shown in FIG. 2, which shows that the composition of the present invention (A 16.0 µM + BO 18.8 µM + BSG 14.2 µM) has a significant synergistic effect on cell growth in high glucose culture (25 mM), but no significant effect on cell proliferation in low glucose culture (5 mM).

### Example 3 Cell Migration Assay

HaCaT cells were cultured at 1 × 10⁵ with addition of 25 mM glucose in a 24-well culture dish for 48 hours, and the following drugs were added to observe the cell migration state:
Con: the control group;
BO 18.8µM +A 16.0µM;
BSG 14.2µM +A 16.0µM;
BO 18.8µM + BSG 14.2µM;
All: A 16.0µM + BO 18.8µM + BSG 14.2µM.

The images of the cell migration results of each group after 48 hours of culture are shown in FIG. 3A. The results of the control group are taken as 1 to compare with other groups in FIG. 3B, which shows that the composition of the present invention (A 16.0µM + BO 18.8µM + BSG 14.2µM) has a promoting effect on cell migration in high glucose culture (25 mM), but BO 18.8 µM, BSG 14.2 µM, or combinations of any two among the three components has no promoting effect. It can be inferred that the composition of the present invention has unexpected therapeutic effects on diabetic wound healing, which further supports its effect on chronic wound healing.

### Example 4 Experimental analysis of inflammatory response

### Analysis of inhibition of IL-1β-induced inflammatory response

Cultured HDF cells (1 × 10⁵/well) were supplemented with the cellular inflammatory factor IL-1β (10 ng/mL) to evaluate the inhibition of IL-1β-induced inflammation by these drugs. Cells were cultured in a 24-well culture dish for 48 hours, and the following drugs were added at the same time to observe how cells in an inflammatory state affected the expression of proteins, including matrix metalloproteinases (such as MMP-1 or MMP-9) up-regulated by inflammation; and expression of collagen (collagen I or Collagen 9) controlled by TGF-β factor down-regulated by inflammation and its downstream signaling pathway:
Con: the control group (without IL-1β treatment);
Con: the control group (IL-1β treatment);
BO : 18.8µM;
BSG: 14.2µM;
A: 16.0µM;
BO +A: BO 18.8µM + A 16.0µM;
BSG +A: BSG 14.2µM + A 16.0µM;
BO +BSG: BO 18.8µM + BSG 14.2µM;
All: BO 18.8µM + BSG 14.2µM +16.0µM.

The results are shown in FIG. 4, showing the effect of each component and its combination on the protein secretion amount under the inflammatory response induced by adding IL-1β. It is shown that the combination of three components has obvious promoting effect on TGF-β factor and its downstream collagen (Collgen I and Collgen III), and has obvious inhibitory effect on MMP-1 induced by inflammation, showing that the three-component combination is far more effective for wound healing than either component or its two-component combination.

### Example 5 Human clinical results

The composition of the present invention (A 16.0 µM + BO 18.8 µM + BSG 14.2 µM) was applied daily to different patients to observe the wound healing. The wound healing results of the three patients are shown in FIGs. 5, 6 and 7 and collated as follows.

### (1) Case 1

Diabetic and stroke patient, male, 77 years old. After treatment, the photos during the healing process of the left heel wound are shown in FIG. 5. On day 0, the original wound size was 4cm × 3cm × 0.5cm (A). After the treatment, the wound started to close obviously on day 18 (B), the wound was reduced to 1.5 cm × 0.5 cm × 0.2 cm on day 33 (C), and the wound was completely healed on day 79 (D), showing that the composition of the present invention has obvious therapeutic effect on diabetic wounds.

### (2) Case 2

Diabetic patient, male, 77 years old. After treatment, the photos during the healing process of the right heel wound are shown in FIG. 6. The original wound size was 15cm × 11 cm × 2 cm on day 0 (A), the wound was reduced to 5.5cm × 11 cm × 1 cm on day 75 after treatment (B), on day 103 the wound healed and the size of the wound was 3cm × 2cm × 0.5 cm (C), and the wound was completely healed on day 140 (D), showing that the composition of the present invention has obvious therapeutic effect on diabetic wounds, and the treatment is ongoing.

### (3) Case 3

Diabetic and lung cancer patient, female, 82 years old, bedridden for a long time with bedsores. After treatment, the photos during the wound healing process are shown in FIG. 7. On day 0, the original wound size was 2.5cm × 1cm × 0.5 cm (A), on day 17 after treatment, the wound started to close obviously and the wound was reduced to 1.5cm × 1 cm × 0.2 cm (B), and the wound was completely healed on day 90 (C), showing that the composition of the present invention has a significant therapeutic effect on bedsores.

Although the present invention is disclosed above with preferred embodiments, it is not intended to limit the present invention. Anyone skilled in the art can make changes and modifications without departing from the spirit and scope of the present invention, of which belong to the protection scope defined by the claims of the present invention.

## Claims

1. A pharmaceutical composition with efficacies of chronic wound healing, mainly composed of the following three components: (1) an anti-inflammatory agent selected from the group consisting of acteoside, isoacteoside and a combination thereof, (2) an astringent selected from the group consisting of gallic acid, subgallic acid, their salts and a combination thereof, and (3) a cooling agent selected from the group consisting of borneal, menthol and a combination thereof and optionally combined with one or more pharmaceutically acceptable carriers; wherein an equivalent ratio of the three components (1) the anti-inflammatory agent: (2) the astringent: (3) the cooling agent is 1:0.1-10:0.1-10.

2. The pharmaceutical composition of claim 1, wherein the chronic wound is a diabetic wound.

3. The pharmaceutical composition of claim 1, wherein the equivalent ratio of the three components (1) the anti-inflammatory agent: (2) the astringent: (3) the cooling agent is 1:0.5-5:0.5-5.

4. The pharmaceutical composition of claim 1, wherein the anti-inflammatory agent is acteoside or an isomer thereof.

5. The pharmaceutical composition of claim 1, wherein the astringent is a salt of gallic acid or subgallic acid.

6. The pharmaceutical composition of claim 5, wherein the salt is a bismuth salt.

7. The pharmaceutical composition of claim 1, wherein the astringent is bismuth subgallate.

8. The pharmaceutical composition of claim 1, wherein the cooling agent is borneal or menthol.

9. The pharmaceutical composition of claim 1, wherein three components of the pharmaceutical composition are (1) acteoside, (2) bismuth subgallate, and (3) borneal.

10. The pharmaceutical composition of claim 9, comprising (1) 0.05%-10% of acteoside, (2) 0.05%-10% of bismuth subgallate, (3) 0.02%-5% of borneal by weight and the pharmaceutically acceptable carrier.

11. The pharmaceutical composition of claim 10, comprising (1) 0.1%-5% of acteoside, (2) 0.06%-6% of bismuth subgallate, (3) 0.03%-3% of borneal by weight and the pharmaceutically acceptable carrier.

12. The pharmaceutical composition of claim 11, comprising (1) 0.3%-5% of acteoside, (2) 0.5%-5% of bismuth subgallate, (3) 0.5%-1% of borneal by weight and the pharmaceutically acceptable carrier.

13. use of a pharmaceutical composition according to any of claims 1 to 12 in the preparation of a medicament for treating a chronic wound.

14. The use of claim 13, wherein the chronic wound is a diabetic wound.

15. The pharmaceutical composition of claim 1, further comprising a suitable excipient for the preparation of an external pharmaceutical form, a cosmetic form or a medicinal material form.
